# EUROPEAN PATENT APPLICATION

(11) **EP 3 757 217 A1**
(43) Date of publication of application: **30.12.2020**
(21) Application number: 19183033.0
(22) Date of filing: 27.06.2019
(51) Int. Cl.: C12N 15/62, C07K 1/18

(54) **METHODS FOR PROTEIN PURIFICATION**

(71) Applicant: GlaxoSmithKline Biologicals S.A., 1330 Rixensart (BE)
(72) Inventor: The designation of the inventor has not yet been filed
(74) Representative: Sanderson, Andrew John

(57) **Abstract**

The present invention relates to methods of protein purification, in particular using ion exchange chromatography. Modified proteins and peptide tags suitable for use in purification by ion exchange chromatography are provided, as are related methods.

## Description

### FIELD OF THE INVENTION

The present invention relates to methods of protein purification, in particular using ion exchange chromatography. Modified proteins and peptide tags suitable for use in purification by ion exchange chromatography are provided, as are related methods.

### BACKGROUND TO THE INVENTION

Production of recombinant proteins requires the proteins to be purified by separating them from the cells in which they are produced, often the most time-consuming and expensive factor in the production process. This is especially true for proteins which are used for medical and therapeutic applications, as a very high level of purity is required. Protein purification usually relies on the combination of several techniques in a multi-step process, starting with cell breakdown removal of cell debris, followed by separation of the desired protein from other cellular proteins and impurities. The amount of material and concentration needed, native folding/activity required, the degree of purity, subunit content of a multimeric protein, the post-translational modifications guide the protein strategy design. In order to design a proper protein purification method, it is crucial to assess protein solubility, its lability at high or low concentrations and its sensitivity to salt concentration, temperature, pH and oxidation. Moreover, when aiming to combine different purification steps, it is desirable to reduce or even abolish any intermediate steps of dialysis and concentration.

Purification usually involves bulk or batch procedures employed early in purification, suitable for large volumes and effective in removing non-protein material (nucleic acids, polysaccharides, and lipids), followed by more refined procedures suitable for obtaining a highly pure product. Bulk procedures include salting out, phase partitioning with organic polymers, precipitation with organic solvents (can lead to denaturation), isoelectric precipitation at very low salt concentration, thermal precipitation and polyethylene glycol (a non-ionic polymer) precipitation. Note that drastic methods such as heat, extreme pH or phase partitioning with organic solvents are suitable only for stable proteins. Precipitation is a rapid, gentle, scalable, and relatively inexpensive method widely used to achieve a substantial enrichment of the target protein due to fractionation and concentration of the target. Ammonium sulphate (AS) and polyethyleneimine (PEI) are the most widely used precipitation agents. AS is stabilizing to protein structures, very soluble, relatively inexpensive and allows protein fractionation exploiting the salting in-salting out phenomenon. In the same line, PEI is a positively charged molecule at neutral pH and it binds to negatively charged macromolecules such as nucleic acid and acidic proteins forming a network that rapidly precipitates.

Refined procedures for purification usually proceed from high to low capacity procedures and include, among others, ion-exchange chromatography, gel filtration, affinity chromatography, hydrophobic interaction chromatography, protein chromatography on hydroxyapatite and Immobilized-metal affinity chromatography. Immobilized-metal affinity chromatography (IMAC) is a technique based on the affinity of transition metal ions such as Zn2+, Cu2+, Ni2+ and Co2+ immobilized on a solid matrix via a strong chelating agent to histidine and cysteine in aqueous solutions. This technique is commonly used with recombinant His-tagged proteins (proteins expressed with an epitope containing six or more histidine residues), which bind to Ni2+ columns. The main advantages of IMAC are its low cost, robustness and simplicity of use, as it also works in denaturing, oxidizing and reducing conditions, with relatively high affinity and specificity. The main limitations include the need to avoid chelating agents (EDTA but also potentially chelating groups such as Tris), the potential immunogenicity of the His tag sequence, the allergenic effects of nickel leaching from an IMAC matrix and the co-purification of contaminant proteins such as proteins with natural metal-binding motifs, proteins with histidine clusters on their surfaces, proteins that bind to heterologously expressed His-tagged proteins, for example by a chaperone mechanism, and proteins with affinity to agarose-based supports. Additionally, IMAC is not suitable for proteins sensitive to metal ions and for proteins susceptible to oxidation or proteolytic damage, as IMAC stationary phase does not tolerate chelating or reducing agents.

Ion exchange chromatography is a versatile method for separation of proteins, frequently used for analytical and preparative purposes. Ion exchange chromatography can achieve a high resolution, with simultaneous purification and concentration of the target.

Ion exchangers are composed of a base matrix, usually porous beads providing a wide adsorption surface, on which a charged ligand, usually a charged polymer to improve the resin's capacity, is immobilized. Exchangers are acid and bases themselves and their degree of protonation on a wide or narrow pH range depends on their being strong or weak acids or bases.

Proteins, polynucleotides, and other biomacromolecules can interact with ion exchangers because they expose charged moieties on their surface, a phenomenon that is dependent on the pH of the solution and on their isoelectric point (pl), which can be estimated based on protein sequence, as long as there are no post-translational modifications. Cation exchangers are negatively charged and bind positively charged proteins below their pI. Anion exchangers are positively charged and bind negatively charged proteins above their pI. Binding of a protein to an ion exchange resin depends not only on the overall charge of the protein but also factors such as charge distribution on the protein surface, which affects the protein binding to the resin which occurs in an oriented manner. Hence, a prediction of protein binding to an ion-exchanger cannot be based on the protein primary structure, and it is not always possible to achieve good binding of a desired protein to an ion exchange resin, particularly at a physiological pH as would be desired in order to maintain proper folding and function. Ion exchange chromatography is useful for separating intact and truncated forms of a protein or protein variants and/or isoforms, which are characterised by the same primary structure but by a different surface structure, reflected by a different retention on ion exchangers; for example, it is possible to separate protein variants which differ by a single charge. This can be done very quickly as ion-exchange chromatography can be operated at room temperature and at linear flow up to 500cm/h, achieving protein separation in less than 5 minutes. However, not all proteins are amenable to easy separation using ion exchange chromatography, as depending on their charge characteristics they may not bind to certain ion exchange resins, or may not bind sufficiently strongly to achieve efficient separation with high yield.

### SUMMARY OF THE INVENTION

The present invention provides fusion proteins comprising a protein of interest and a peptide tag. Preferably, the peptide tag is able to bind to an ion exchange resin, in particular a cation exchange resin. The peptide tag serves to enhance binding of the protein to ion exchange resins and facilitate purification of the proteins purified by ion exchange chromatography. Peptide tags such as His-tags are known in the art, for use in affinity chromatography on metal ion columns (e.g. IMAC). However, the present inventors have found that peptide tags may also be used to permit or optimise purification of proteins by ion exchange chromatography. Tags effective for this purpose have been developed and are disclosed herein.

The invention thus provides a fusion protein suitable for purification via ion exchange chromatography, which protein comprises (i) a protein of interest, and (ii) a peptide tag at the N or C terminus. The tag suitably comprises or consists of Rₙ, (HR)ₙ, (PR)ₙ, (SR)ₙ or (PSR)ₙ, where 'n' is preferably an integer from 2 to 6 inclusive.

Also provided is a fusion protein comprising (i) a protein of interest, and (ii) a peptide tag at the N or C terminus, which tag comprises or consists of Rₙ, (HR)ₙ, (PR)ₙ, (SR)ₙ or (PSR)ₙ, where 'n' is preferably an integer from 2 to 6 inclusive.

Also provided is a fusion protein comprising a protein of interest covalently linked directly or indirectly to a peptide tag which is capable of binding to an ion exchange resin. The tag suitably comprises or consists of Rₙ, (HR)ₙ, (PR)ₙ, (SR)ₙ or (PSR)ₙ, where 'n' is preferably an integer from 2 to 6 inclusive.

The peptide tag suitably is from 2 to 20 amino acids in length, preferably from 4 to 12 amino acids in length. Preferably, the tag comprises charged amino acids. The tag may also comprise one or more proline residues. In an embodiment, the tag comprises or consists of an amino acid sequence of any one of SEQ ID Nos 1 to 9.

In an embodiment, the tag is not a His tag, i.e. does not comprise Hₙ where 'n' is ≥2. In an embodiment, the tag is not a His₆ tag. In the context of a vaccine antigen, using a tag which is not a His tag reduces the risk of inducing or being the target of antibodies which cross-react with His-tagged proteins, which are commonly produced and purified by affinity chromatography.

The fusion protein may further comprise a linker between the protein of interest and the peptide tag. The linker may be a short peptide sequence of 1, 2 or 3 amino acids and may advantageously comprise amino acids with a moderate to high degree of freedom, providing a flexible linker, such as G or S. In an embodiment the linker comprises GG, GS, SS or SG.

The protein of interest may be an antigenic protein, such as a vaccine antigen, and/or a carrier protein for conjugation to a polysaccharide. Typical carrier proteins include tetanus toxoid (TT), diphtheria toxoid (DT), CRM₁₉₇, AcrAfrom *C*. *jejuni,* protein D from *Haemophilus influenzae,* exotoxin A of *Pseudomonas aeruginosa* (EPA), detoxified pneumolysin from *Streptococcus. pneumoniae,* meningococcal outer membrane protein complex (OMPC). Bacterial vaccine antigens such as detoxified Hla from *S. aureus* or ClfA from S. *aureus* may also be used as carrier proteins.

In an embodiment, the protein of interest is exotoxin A from *Pseudomonas aeruginosa* (EPA). Said EPA may comprise the amino acid sequence of SEQ ID NO. 10 or an amino acid sequence at least 80%, 85%, 90%, 92%, 95%, 96%, 97%, 98% or 99% identical to SEQ ID NO. 10. The EPA protein may be modified in that it comprises a L to V substitution at the amino acid position corresponding to position L552 of SEQ ID NO. 10, and/or deletion of E553 of SEQ ID NO: 10, or at equivalent positions within an amino acid sequence at least 80%, 85%, 90%, 92%, 95%, 96%, 97%, 98% or 99% identical to SEQ ID NO. 10 (e.g. SEQ ID NO: 11); and/or one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline, which substitution is optionally substitution of A375, A376 or K240 of SEQ ID NO: 10 with K-D-Q-N-R-T-K (SEQ ID NO: 27) or K-D-Q-N-A-T-K (SEQ ID NO: 28). Hence, the protein of interest may comprise the amino acid sequence of SEQ ID NO: 11 or an amino acid sequence at least 80%, 85%, 90%, 92%, 95%, 96%, 97%, 98% or 99% identical to SEQ ID NO. 11, optionally with insertion or substitution of one or more amino acids with K-D-Q-N-R-T-K (SEQ ID NO: 27) or K-D-Q-N-A-T-K (SEQ ID NO: 28.

In an embodiment, the protein of interest is Hla from *Staphylococcus aureus.* In an embodiment, said Hla comprises the amino acid sequence of SEQ ID NO: 19 or an amino acid sequence at least 80%, 85%, 90%, 92%, 95%, 96%, 97%, 98% or 99% identical to SEQ ID NO. 19. The Hla protein may be modified in that the amino acid sequence comprises an amino acid substitution at position H35 of SEQ ID NO. 19 or at an equivalent position within an amino acid sequence at least 80%, 85%, 90%, 92%, 95%, 96%, 97%, 98% or 99% identical to SEQ ID NO. 19, which substitution is optionally H35L. The Hla protein may be modified in that one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline. In an embodiment, said substitution is substitution of K131 of SEQ ID NO: 19 with K-D-Q-N-R-T-K (SEQ ID NO: 27). The Hla protein may be modified in that the amino acid sequence comprises amino acid substitutions at positions H48 and G122 of SEQ ID NO. 1 or at equivalent positions within an amino acid sequence at least 80%, 85%, 90%, 92%, 95%, 96%, 97%, 98% or 99% identical to SEQ ID NO. 19. In an embodiment, said substitutions are respectively H to C and G to C.

In an embodiment, the fusion protein comprises (i) an EPA protein as disclosed herein, and (ii) a peptide tag consisting or comprising of any one of SEQ ID Nos: 1-9. In a preferred embodiment, said peptide tag comprises or consists of any one of SEQ ID Nos: 6, 8 and 9. In a preferred embodiment, said peptide tag comprises or consists of SEQ ID NO: 8.

In an embodiment, the fusion protein comprises (i) an Hla protein as disclosed herein, and (ii) a peptide tag consisting or comprising of any one of SEQ ID Nos: 1-9. In a preferred embodiment, said peptide tag comprises or consists of any one of SEQ ID Nos: 1-4. In a preferred embodiment, said peptide tag comprises or consists of any one of SEQ ID Nos: 1-3.

In an embodiment, the fusion protein comprises the amino acid sequence of any one of SEQ ID NOs: 12 to 18. In an embodiment, the fusion protein comprises the amino acid sequence of any one of SEQ ID NOs: 14, 17 or 18. In an embodiment, the fusion protein comprises the amino acid sequence of any one of SEQ ID NOs: 12 to 18, modified in that one or more amino acids are substituted with K-D-Q-N-R-T-K (SEQ ID NO: 27) or K-D-Q-N-A-T-K (SEQ ID NO: 28). In an embodiment, the fusion protein comprises the amino acid sequence of any one of SEQ ID NOs: 14, 17 or 18, , modified in that one or more amino acids are substituted with K-D-Q-N-R-T-K (SEQ ID NO: 27) or K-D-Q-N-A-T-K (SEQ ID NO: 28). In an embodiment, the fusion protein comprises the amino acid sequence of any one of SEQ ID NOs: 21 to 23. In an embodiment, the fusion protein comprises the amino acid sequence of any one of SEQ ID NOs: 21 or 22. In an embodiment, the fusion protein does not comprise the amino acid sequence of SEQ ID NO: 24.

In one aspect, the invention provides a method of purifying a fusion protein of the invention, or a conjugate of the invention, or a bioconjugate of the invention, the method comprising a step of ion exchange chromatography. In an embodiment, a step of ion exchange chromatography will involve the steps of
(i) binding the fusion protein to an ion exchange resin using a loading buffer,
(ii) washing the ion exchange resin using a washing buffer, and
(iii) eluting the protein from the ion exchange resin using an elution buffer.

In one aspect, the invention provides a method of purifying a protein of interest, the method comprising (i) producing a fusion protein comprising the protein of interest and a peptide tag which binds to an ion exchange resin, and (ii) purifying the fusion protein by ion exchange chromatography. Suitable peptide tags are disclosed herein.

In one aspect, the invention provides a method of purification of a protein of interest comprising subjecting the protein to ion exchange chromatography, wherein the protein has been modified by addition of a peptide tag as disclosed herein at the N or C terminus. Suitable peptide tags are disclosed herein.

In one aspect, the invention provides a conjugate (e.g. bioconjugate) comprising a polysaccharide, e.g. a polysaccharide antigen, linked, e.g. covalently linked, to a protein of interest as disclosed herein.

The invention also provides a conjugate (e.g. bioconjugate) comprising a polysaccharide, e.g. a polysaccharide antigen, linked, e.g. covalently linked, to a fusion protein of the invention.

In one aspect, the invention provides a polynucleotide encoding a fusion protein of the invention.

In one aspect, the invention provides a vector comprising a polynucleotide encoding a fusion protein of the invention.

In one aspect, the invention provides an immunogenic composition comprising a fusion protein of the invention, or a conjugate of the invention, or a bioconjugate of the invention and a pharmaceutically acceptable excipient or carrier.

In one aspect, the invention provides a vaccine comprising a fusion protein of the invention, or a conjugate of the invention, or a bioconjugate of the invention and a pharmaceutically acceptable excipient or carrier.

In one aspect, the invention provides a pharmaceutical composition comprising a fusion protein of the invention and a pharmaceutically acceptable excipient or carrier.

In one aspect, the invention provides a method of making an immunogenic composition of the invention comprising the step of mixing the fusion protein or the conjugate or the bioconjugate of the invention with a pharmaceutically acceptable excipient or carrier.

In one aspect, the invention provides a method of immunising a human host comprising administering to the host a fusion protein of the invention, or a conjugate of the invention, or a bioconjugate of the invention.

In one aspect, the invention provides a method of inducing an immune response to an antigen, for example a protein of interest as described herein, in a subject, the method comprising administering to said subject a therapeutically or prophylactically effective amount of a fusion protein of the invention, or a conjugate of the invention, or a bioconjugate of the invention.

In one aspect, the invention provides a fusion protein of the invention, or a conjugate of the invention, or a bioconjugate of the invention for use in a method of medical treatment or prevention.

### DESCRIPTION OF THE FIGURES

FIGURE 1 Purification on Nuvia-S cation exchange column of Hla-CP5 tagged with HHHH, RRRR, HHRR and HRHR peptides (SDS-PAGE).
FIGURE 2: Purification on cation exchange column of CP5-Hla carrying a C-terminal HRHR tag (Western blot with anti-Hla antibody). Gel A: 40 microlitre loaded. Gel B: 20 microlitre loaded.
FIGURE 3: Purification on cation exchange column of non-tagged CP5-Hla. The same procedure as for Fig 2 was carried out using non-tagged CP5-Hla. Gel A: 20 microlitre loaded. Gel B: 40 microlitre loaded.
FIGURE 4: Purification on Capto S cation exchange column of EPA-Sp33F tagged with HRHR peptide (Western blot with anti-EPA antibody).
FIGURE 5: Purification on Capto S cation exchange column of EPA-Sp33F tagged with HRHRHR peptide (SDS-PAGE).
FIGURE 6: Purification on Capto S cation exchange column of EPA-Sp33F tagged with HRHRHRHR peptide (Western blot with anti-EPA antibody).
FIGURE 7: Purification on cation exchange column of EPA-Sp33F tagged with RRRR peptide (Western blot with anti-EPA antibody)
FIGURE 8: Purification on Capto S cation exchange column of EPA-Sp33F tagged with RRRRRR peptide (Western blot with anti-EPA antibody).
FIGURE 9: Purification on Capto S cation exchange column of EPA-Sp33F tagged with PRPRPRPRPRPR peptide (Western blot with anti-EPA antibody).
FIGURE 10: Purification on Capto S cation exchange column of EPA-Sp33F tagged with PSRPSRPSRPSR peptide (Western blot with anti-EPA antibody).
Figure 11: Purification on Capto S cation exchange column of EPA-Sp8 tagged with PRPRPRPRPRPR peptide (Western blot with anti-EPA antibody).
Figure 12: Purification on Capto S cation exchange column of EPA-Sp2 tagged with PRPRPRPRPRPR peptide (Western blot with anti-EPA antibody).

### DETAILED DESCRIPTION OF THE INVENTION

### DEFINITIONS

Peptide tag: As used herein, the term 'peptide tag' refers to a short (preferably 2-20 amino acids) amino acid sequence which is fused to the N- or C-terminus of a protein of interest.

Tagged protein: As used herein, a 'tagged protein' refers to a polypeptide comprising the protein of interest with a peptide tag fused to the N or C terminus. The tagged protein may also comprise an amino acid linker, preferably of one or two amino acids, between the protein and the peptide tag.

Fusion protein: As used herein, the term "fusion protein" refers to a protein comprising amino acid sequence from different polypeptides. Conveniently, they may be encoded by a single nucleotide sequence encoding the two or more amino acid sequences, for example a single nucleotide sequence containing 2 or more genes or genes, portions of genes or other nucleotide sequence encoding a peptide or polypeptide.

A used herein, the term "carrier protein" refers to a protein covalently attached to a polysaccharide antigen (e.g. saccharide antigen) to create a conjugate (e.g. bioconjugate). A carrier protein activates T-cell mediated immunity in relation to the polysaccharide antigen to which it is conjugated.

As used herein, the term "bioconjugate" refers to conjugate between a protein (*e.g.* a carrier protein) and an antigen (e.g. a saccharide) prepared in a host cell background, wherein host cell machinery links the antigen to the protein (*e.g.* N-links).

As used herein, the term "glycosite" refers to an amino acid sequence recognized by a bacterial oligosaccharyltransferase, e.g. PgIB of *C. jejuni.* The minimal consensus sequence for PgIB is D/E-X-N-Z-S/T (SEQ ID NO. 25), while the extended consensus sequence K-D/E-X-N-Z-S/T-K (SEQ ID NO. 26) may also be used.

Any amino acid apart from proline (pro, P): refers to an amino acid selected from the group consisting of alanine (ala, A), arginine (arg, R), asparagine (asn, N), aspartic acid (asp,D), cysteine (cys, C), glutamine (gln, Q), glutamic acid (glu, E), glycine (gly, G), histidine (his, H), isoleucine (ile,I), leucine (leu, L), lysine (lys, K), methionine (met, M), phenylalanine (phe, F), serine (ser, S), threonine (thr, T), tryptophan (trp, W), tyrosine (tyr, Y), valine (val, V).
EPA: exotoxin A of *Pseudomonas aeruginosa.*
Hla: Haemolysin A, also known as alpha toxin, from a staphylococcal bacterium, in particular *S. aureus.*
CP: Capsular polysaccharide.

As used herein, the term "effective amount," in the context of administering a therapy (*e.g.* an immunogenic composition or vaccine of the invention) to a subject refers to the amount of a therapy which has a prophylactic and/or therapeutic effect(s).

As used herein, the term "subject" refers to an animal, in particular a mammal such as a primate (e.g. human).

As used herein, reference to a percentage sequence identity between two amino or nucleic acid sequences means that, when aligned, that percentage of amino acids or bases are the same in comparing the two sequences. This alignment and the percent homology or sequence identity can be determined using software programs known in the art, for example those described in section 7.7.18 of Current Protocols in Molecular Biology (F.M. Ausubel et al., eds., 1987, Supplement 30). A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in Smith & Waterman (1981) Adv. Appl. Math. 2: 482-489. Percentage identity to any particular sequence (*e.g.* to a particular SEQ ID) is ideally calculated over the entire length of that sequence. The percentage sequence identity between two sequences of different lengths is preferably calculated over the length of the longer sequence. Global or local alignments may be used. Preferably, a global alignment is used.

As used herein, the term "purifying" or "purification" of a fusion protein or protein of interest, or conjugate (eg bioconjugate) thereof, means separating it from one or more contaminants. A contaminant is any material that is different from said fusion protein or protein of interest, or conjugate (eg bioconjugate) thereof. Contaminants may be, for example, cell debris, nucleic acid, lipids, proteins other than the fusion protein or protein of interest, polysaccharides and other cellular components.

A "recombinant" polypeptide is one which has been produced in a host cell which has been transformed or transfected with nucleic acid encoding the polypeptide, or produces the polypeptide as a result of homologous recombination.

As used herein, the term "conservative amino acid substitution" involves substitution of a native amino acid residue with a non-native residue such that there is little or no effect on the size, polarity, charge, hydrophobicity, or hydrophilicity of the amino acid residue at that position, and without resulting in decreased immunogenicity. For example, these may be substitutions within the following groups: valine, glycine; glycine, alanine; valine, isoleucine, leucine; aspartic acid, glutamic acid; asparagine, glutamine; serine, threonine; lysine, arginine; and phenylalanine, tyrosine. Conservative amino acid modifications to the sequence of a polypeptide (and the corresponding modifications to the encoding nucleotides) may produce polypeptides having functional and chemical characteristics similar to those of a parental polypeptide.

As used herein, the term "deletion" is the removal of one or more amino acid residues from the protein sequence. Typically, no more than about from 1 to 6 residues (e.g. 1 to 4 residues) are deleted at any one site within the protein molecule.

As used herein, the term "insertion" is the addition of one or more non-native amino acid residues in the protein sequence. Typically, no more than about from 1 to 6 residues (e.g. 1 to 4 residues) are inserted at any one site within the protein molecule.

As used herein, the term 'comprising' indicates that other components in addition to those named may be present, whereas the term 'consisting of' indicates that other components are not present, or not present in detectable amounts. The term 'comprising' naturally includes the term 'consisting of'.

### STATEMENT OF THE INVENTION

### Peptide tag

Peptide tags as used with the present invention bind to ion exchange resins, in particular cationic exchange resins. The tags thus suitably include charged amino acid residues, such as K, R, H, D and E. Where the tag is intended for binding to a cationic exchange resin, K, R, H, particularly H and R, are preferred. Residues such as proline may also be included to improve the accessibility of the charged residues in the tag.

The skilled person will understand that the amino acid composition and length of the tag may be adapted to optimise binding to ion exchange resin depending on the size, amino acid composition, charge and charge accessibility of the protein of interest. For example, the longer the tag, the more strongly it will bind to the resin, so a longer tag may be required for a protein which has only a low overall charge at a given pH.

In an embodiment, a peptide tag may be 2, 3, 4, 5, 6, 7, 8, 9, 10, 11, 12, 13, 14, 15, 16, 17, 18, 19, 20 or more amino acids in length. Preferably, the tag is between 4 and 12 amino acids in length.

Exemplary tags include Rₙ, Hₙ, (HR)ₙ, (PR)ₙ, (SR)ₓ, (PSR)ₙ, where 'x' is preferably an integer from one to 10, for example 1, 2, 3, 4, 5, 6, 7, 8, 9 or 10. A suitable tag may be HHHH, RRRR, RRRRRR, HRHR, HRHRHR, HRHRHRHR, (PR)₆ or (PSR)₄. (PR)ₙ, where 'n' is 2, 3, 4, 5, or 6, is a particularly suitable tag.

In an embodiment, the tag is not HRHR. In an embodiment, the tag does not comprise HRHR. In a specific embodiment, the tagged protein is Hla and the tag is not HRHR. In a specific embodiment, the tagged protein does not comprise the amino acid sequence of SEQ ID No 24.

In an embodiment, the tag is not a His-tag. In an embodiment, the tag is not a His₆ tag. In the context of a vaccine antigen, using a tag which is not a His tag reduces the risk of inducing or being the target of antibodies which cross-react with His-tagged proteins, which are commonly produced and purified by affinity chromatography.

### Protein of interest

The protein of interest may be any protein, in particular a recombinant protein. In an embodiment, the protein is an antigenic protein, for example a vaccine antigen. In an embodiment, the protein is for use as a carrier protein for a polysaccharide antigen. A carrier protein may be, for example, tetanus toxoid (TT), diphtheria toxoid (DT), CRM₁₉₇, AcrA from *C. jejuni,* exotoxin A of *Pseudomonas aeruginosa* (EPA), protein D from *Haemophilus influenzae,* detoxified pneumolysin from *Streptococcus. pneumoniae,* meningococcal outer membrane protein complex (OMPC). Bacterial vaccine antigens such as detoxified Hla from *S. aureus* or ClfA from *S. aureus* may also be used as carrier proteins.

In a specific embodiment, the protein of interest is Exotoxin A of *Pseudomonas aeruginosa* (EPA). EPA is a 67 kDa extracellularly secreted protein comprising 613 amino acids in its mature form. The protein may be detoxified, for example by mutating/deleting the catalytically essential residues L552VΔE553, as described in Lukac et al, Infect Immun, 56, 3095-3098, 1988 and Ho et al, Hum Vaccin, 2, 89-98, 2006. Where the protein is to be used as a carrier in a bioconjugate, one or more PgIB consensus sequences may be engineered into the protein, as described below. Additionally, to enable its glycosylation in *E. coli,* it may be useful to include a signal peptide which the protein must locate to the periplasmic space for glycosylation to occur, as described below.

In an embodiment, the protein of interest may be an EPA sequence comprising or consisting of an amino acid sequence of SEQ ID NO. 10 or an amino acid sequence at least 80%, 85%, 90%, 92%, 95%, 96%, 97%, 98% or 99% identical to SEQ ID NO. 10. In an embodiment, the protein of interest comprises or consists of an amino acid sequence at least 80%, 85%, 90%, 92%, 95%, 96%, 97%, 98% or 99% identical to SEQ ID NO. 10, modified in that the amino acid sequence comprises a non-conservative amino acid substitution (for example, L to V) at position L552 and deletion of residue E553, wherein said positions correspond to positions L552 and E553 of SEQ ID NO. 10 or equivalent positions within an amino acid sequence at least 80%, 85%, 90%, 92%, 95%, 96%, 97%, 98% or 99% identical to SEQ ID NO. 10 (e.g. SEQ ID NO: 11).

Said modified EPA protein may be further modified to comprise one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline (e.g. SEQ ID NO: 28), also referred to herein as a 'glycosite'. In an embodiment, said consensus sequence is substituted for an amino acid residue within said EPA sequence. Accordingly, the protein of interest may be an EPA protein comprising an amino acid sequence of SEQ ID NO. 10 or an amino acid sequence at least 80%, 85%, 90%, 92%, 95%, 96%, 97%, 98% or 99% identical to SEQ ID NO. 10, modified in that the amino acid sequence comprises one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline. In an embodiment, said consensus sequence is substituted for A375, A376 or K240 of SEQ ID NO: 10 or an amino acid sequence at least 80%, 85%, 90%, 92%, 95%, 96%, 97%, 98% or 99% identical to SEQ ID NO. 10. In an embodiment, said modified EPA protein contains the following mutations: L552V/ΔE553, and substitution of one or more amino acids with glycosite KDQNATK.

Hence, for example, the fusion protein may comprise or consist of the amino acid sequence of SEQ ID NO: 10 or SEQ ID NO:11, or an amino acid sequence at least 80%, 85%, 90%, 92%, 95%, 96%, 97%, 98% or 99% identical to SEQ ID NO: 10 or SEQ ID NO: 11, and a peptide tag comprising or consisting of the amino acid sequence of any one of SEQ ID Nos: 1-9. In an embodiment, the fusion protein may comprise or consist of the amino acid sequence of SEQ ID NO: 10 or SEQ ID NO:11, or an amino acid sequence at least 80%, 85%, 90%, 92%, 95%, 96%, 97%, 98% or 99% identical to SEQ ID NO: 10 or SEQ ID NO: 11, and a peptide tag comprising or consisting of the amino acid sequence of any one of SEQ ID Nos: 6, 8 or 9. In a preferred embodiment, the fusion protein may comprise or consist of the amino acid sequence of SEQ ID NO: 10 or SEQ ID NO:11, or an amino acid sequence at least 80%, 85%, 90%, 92%, 95%, 96%, 97%, 98% or 99% identical to SEQ ID NO: 10 or SEQ ID NO: 11, and a peptide tag comprising or consisting of the amino acid sequence of any one of SEQ ID No 8 or SEQ ID NO: 9. In a particularly preferred embodiment, the fusion protein may comprise or consist of the amino acid sequence of SEQ ID NO: 10 or SEQ ID NO:11, or an amino acid sequence at least 80%, 85%, 90%, 92%, 95%, 96%, 97%, 98% or 99% identical to SEQ ID NO: 10 or SEQ ID NO: 11, and a peptide tag comprising or consisting of the amino acid sequence of SEQ ID No 8. In specific embodiments, the fusion protein comprises or consists of the amino acid sequence of any one of SEQ ID NO: 12-18, optionally with insertion of one or more glycosites as described herein. In specific embodiments, the fusion protein comprises or consists of the amino acid sequence of any one of SEQ ID NOs: 14, 17 or 18, optionally with insertion of one or more glycosites as described herein. In a preferred embodiment, the fusion protein comprises the sequence of SEQ ID NO: 17 or SEQ ID NO: 18, optionally with insertion of one or more glycosites as described herein. In a particularly preferred embodiment, the fusion protein comprises the sequence of SEQ ID NO: 17, optionally with insertion of one or more glycosites as described herein.

In a specific embodiment, the protein of interest is Hla.

In an embodiment, the protein of interest may be an Hla sequence comprising or consisting of an amino acid sequence of SEQ ID NO. 19 or an amino acid sequence at least 80%, 85%, 90%, 92%, 95%, 96%, 97%, 98% or 99% identical to SEQ ID NO. 19. In an embodiment, the protein of interest comprises or consists of an amino acid sequence at least 80%, 85%, 90%, 92%, 95%, 96%, 97%, 98% or 99% identical to SEQ ID NO. 19, modified in that the amino acid sequence comprises amino acid substitutions at positions H48 and G122 of SEQ ID NO. 19 or at equivalent positions within an amino acid sequence at least 80%, 85%, 90%, 92%, 95%, 96%, 97%, 98% or 99% identical to SEQ ID NO. 19, wherein said substitutions are respectively H to C and G to C (e.g. SEQ ID NO: 20).

Said modified Hla protein may be further modified in that the amino acid sequence comprises an amino acid substitution at position H35 (e.g. H35L) of SEQ ID NO. 19 or at an equivalent position within an amino acid sequence at least 80%, 85%, 90%, 92%, 95%, 96%, 97%, 98% or 99% identical to SEQ ID NO. 19 (e.g. SEQ ID NO: 20). Said modified Hla protein may be further modified to comprise one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline (e.g. SEQ ID NO: 27). In an embodiment, said modified Hla protein contains the following mutations: H35L, H48C and G122C, and a glycosite KDQNRTK substituted for K131 of SEQ ID NO: 1 (for example, SEQ ID Nos: 20-24). Accordingly, the protein of interest may be an Hla protein comprising an amino acid sequence of SEQ ID NO. 19 or an amino acid sequence at least 80%, 85%, 90%, 92%, 95%, 96%, 97%, 98% or 99% identical to SEQ ID NO. 19, modified in that the amino acid sequence comprises one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline.

Hence, for example, the fusion protein may comprise or consist of the amino acid sequence of SEQ ID NO: 19 or SEQ ID NO:20, or an amino acid sequence at least 80%, 85%, 90%, 92%, 95%, 96%, 97%, 98% or 99% identical to SEQ ID NO: 19 or SEQ ID NO: 20, and a peptide tag comprising or consisting of the amino acid sequence of any one of SEQ ID Nos: 1-9. In an embodiment, the fusion protein may comprise or consist of the amino acid sequence of SEQ ID NO: 19 or SEQ ID NO:20, or an amino acid sequence at least 80%, 85%, 90%, 92%, 95%, 96%, 97%, 98% or 99% identical to SEQ ID NO: 19 or SEQ ID NO: 20, and a peptide tag comprising or consisting of the amino acid sequence of any one of SEQ ID Nos: 1-4. In an embodiment, the fusion protein may comprise or consist of the amino acid sequence of SEQ ID NO: 19 or SEQ ID NO:20, or an amino acid sequence at least 80%, 85%, 90%, 92%, 95%, 96%, 97%, 98% or 99% identical to SEQ ID NO: 19 or SEQ ID NO: 20, and a peptide tag comprising or consisting of the amino acid sequence of any one of SEQ ID Nos: 1-3. In an embodiment, the fusion protein may comprise or consist of the amino acid sequence of SEQ ID NO: 19 or SEQ ID NO:20, or an amino acid sequence at least 80%, 85%, 90%, 92%, 95%, 96%, 97%, 98% or 99% identical to SEQ ID NO: 19 or SEQ ID NO: 20, and a peptide tag comprising or consisting of the amino acid sequence of any one of SEQ ID Nos: 1 or 3. In specific embodiments, the fusion protein comprises or consists of the amino acid sequence of any one of SEQ ID NO: 21-24. In specific embodiments, the fusion protein comprises or consists of the amino acid sequence of any one of SEQ ID NO: 21-23. In a preferred embodiment, the fusion protein comprises the sequence of SEQ ID NO: 23. In an embodiment, the fusion protein does not comprise the sequence of SEQ ID NO: 24.

The protein of interest may further comprise a signal sequence at the N-terminus, for example a signal sequence which is capable of directing the Hla protein to the periplasm of a host cell (e.g. bacterium). This is of particular utility where the protein of interest is a carrier protein intended for use in a bioconjugate. In specific embodiments, the signal sequence may be from *E*. *coli* flagellin (Flgl) [MIKFLSALILLLVTTAAQA (Seq ID NO. 29)], *E. coli* outer membrane porin A (OmpA) [MKKTAIAIAVALAGFATVAQA (Seq ID NO. 30)], *E. coli* maltose binding protein (MalE) [MKIKTGARILALSALTTMMFSASALA (Seq ID NO. 31)], *Erwinia carotovorans* pectate lyase (PeIB) [MKYLLPTAAAGLLLLAAQPAMA (Seq ID NO. 32)], heat labile *E. coli* enterotoxin LTIIb [MSFKKIIKAFVIMAALVSVQAHA (Seq ID NO. 33)], *Bacillus subtilis* endoxylanase XynA [MFKFKKKFLVGLTAAFMSISMFSATASA (Seq ID NO. 34)], *E. coli* DsbA [MKKIWLALAGLVLAFSASA (Seq ID NO. 35)], TolB [MKQALRVAFGFLILWASVLHA (Seq ID NO. 36)] or SipA [MKMNKKVLLTSTMAASLLSVASVQAS (SEQ ID NO.37)]. Where the protein of interest is EPA, in particular an EPA protein as described herein, the signal sequence may be DsbA (SEQ ID NO: 35). Where the protein of interest is Hla, in particular a Hla protein as described herein, the signal sequence may be Flgl (SEQ ID NO: 29).

### Conjugates

In some embodiments, the protein of interest is conjugated to a polysaccharide to form a conjugate. In the context of a vaccine, conjugation of an antigenic polysaccharide to a protein carrier is required for protective memory response, as polysaccharides are T-cell independent antigens. Polysaccharides may be conjugated to protein carriers by different chemical methods, using activation reactive groups in the polysaccharide as well as the protein carrier, and by bioconjugation methods exploiting the enzymes which couple bacterial polysaccharides to proteins.

In an embodiment, the conjugate comprises a conjugate comprising (or consisting of) a protein of interest as disclosed herein covalently linked to a polysaccharide antigen, wherein the antigen is linked (either directly or through a linker) to an amino acid residue of said protein.

In an embodiment, the conjugate comprises a conjugate comprising (or consisting of) a fusion protein of the invention covalently linked to a polysaccharide antigen, wherein the antigen is linked (either directly or through a linker) to an amino acid residue of the fusion protein.

In an embodiment, the conjugate is a bioconjugate. In an embodiment, the conjugate is a chemical conjugate. In an embodiment, the antigen in a conjugate (e.g. bioconjugate) of the invention is a saccharide such as a bacterial capsular saccharide, a bacterial lipopolysaccharide or a bacterial oligosaccharide. In an embodiment the antigen is a bacterial capsular saccharide.

Bacterial capsular saccharides may be, for example: *N. meningitidis* serogroup A capsular saccharide (MenA), *N. meningitidis* serogroup C capsular saccharide (MenC), *N. meningitidis* serogroup Y capsular saccharide (MenY), *N. meningitidis* serogroup W capsular saccharide (MenW), *H. influenzae* type b capsular saccharide (Hib), Group B Streptococcus group I capsular saccharide, Group B Streptococcus group II capsular saccharide, Group B Streptococcus group III capsular saccharide, Group B Streptococcus group IV capsular saccharide, Group B Streptococcus group V capsular saccharide, *Staphylococcus aureus* type 5 capsular saccharide, *Staphylococcus aureus* type 8 capsular saccharide, Vi saccharide from *Salmonella typhi, N. meningitidis* LPS (such as L3 and/or L2), *M. catarrhalis* LPS, *H. influenzae* LPS, Shigella O-antigens, *P.aeruginosa* O-antigens, *E. coli* O-antigens or *S*. *pneumoniae* capsular polysaccharide.

In an embodiment, the protein of interest is linked the polysaccharide via a bioconjugation approach. Briefly, the approach involves in vivo production of glycoproteins in bacterial cells, for example, Gram-negative cells such as *E. coli.* The polysaccharides are assembled on carrier lipids from common precursors (activated sugar nucleotides) at the cytoplasmic membrane by different glycosyltransferases with defined specificity. The synthesis of polysaccharides starts with the addition of a monosaccharide to the carrier lipid undecaprenyl phosphate at the cytoplasmic side of the membrane. The antigen is built up by sequential addition of monosaccharides from activated sugar nucleotides by different glycosyltransferases and the lipid-linked polysaccharide is flipped through the membrane by a flippase. The antigen-repeating unit is polymerized by an enzymatic reaction. The polysaccharide is then transferred to the lipid by a ligase and exported to the periplasm. At the periplasm, the polysaccharides may be linked (e.g. N-linked) to a protein carrier using bacterial oligosaccharyl transferases such as PgIB from *Campylobacter jejuni.*

N-linked protein glycosylation - the addition of carbohydrate molecules to an asparagine residue in the polypeptide chain of the target protein - commonly occurs in eukaryotic organisms. In eukaryotes, the process is accomplished by the enzymatic oligosaccharyltransferase complex (OST) responsible for the transfer of a preassembled oligosaccharide from a lipid carrier (dolichol phosphate) to an asparagine residue of a nascent protein within the conserved sequence Asn-X-Ser/Thr (where X is any amino acid except proline) in the endoplasmic reticulum. The food-borne pathogen *Campylobacter jejuni* can also N-glycosylate iproteins (Wacker et al. Science. 2002; 298(5599):1790-3) using glycosylation machinery encoded by a cluster called "pgl" (for protein glycosylation). The C. *jejuni* glycosylation machinery can be transferred to *E. coli* to allow for the glycosylation of recombinant proteins expressed by the *E. coli* cells. Previous studies have demonstrated how to generate *E. coli* strains that can perform N-glycosylation (see, e.g. Wacker et al. Science. 2002; 298 (5599):1790-3; Nita-Lazar et al. Glycobiology. 2005; 15(4):361-7; Feldman et al. Proc Natl Acad Sci U S A. 2005; 102(8):3016-21; Kowarik et al. EMBO J. 2006; 25(9):1957-66; Wacker et al. Proc Natl Acad Sci U S A. 2006; 103(18):7088-93; International Patent Application Publication Nos. WO2003/074687, WO2006/119987, WO 2009/104074, and WO/2011/06261, and WO2011/138361). Production of bioconjugates is also described in detail in, for example, International Patent Application No. PCT/EP2013/068737 (published as WO 14/037585) and International Patent Application No. PCT/EP2018/085854.

Thus, host cells used to produce bioconjugates are engineered to comprise heterologous nucleic acids, e.g. heterologous nucleic acids that encode one or more carrier proteins and/or heterologous nucleic acids that encode one or more proteins, e.g. genes encoding one or more proteins. Heterologous nucleic acids that encode proteins involved in glycosylation pathways (e.g. prokaryotic and/or eukaryotic glycosylation pathways) may be introduced into the host cells of the invention. Such nucleic acids may encode proteins including oligosaccharyl transferases, epimerases, flippases, polymerases, and/or glycosyltransferases.

The invention thus provides a host cell comprising:
i) one or more nucleic acids that encode glycosyltransferase(s);
ii) a nucleic acid that encodes an oligosaccharyl transferase;
iii) a nucleic acid that encodes a fusion protein of the invention; and optionally
iv) a nucleic acid that encodes a polymerase (*e.g. wzy*).

Also provided is a process for producing a bioconjugate that comprises (or consists of) a fusion protein of the invention linked to a saccharide, said method comprising: (i) culturing a host cell of the invention under conditions suitable for the production of proteins and (ii) isolating the bioconjugate produced by said host cell.

In another embodiment, the protein of interest is covalently linked to the polysaccharide through a chemical linkage obtainable using a chemical conjugation method (i.e. the conjugate is produced by chemical conjugation).

In an embodiment, the chemical conjugation method is selected from the group consisting of carbodiimide chemistry, reductive animation, cyanylation chemistry (for example CDAP chemistry), maleimide chemistry, hydrazide chemistry, ester chemistry, and N-hydroysuccinimide chemistry. Conjugates can be prepared by direct reductive amination methods as described in, US200710184072 (Hausdorff) US 4365170 (Jennings) and US 4673574 (Anderson). Other methods are described in EP-0-161-188, EP-208375 and EP-0-477508. The conjugation method may alternatively rely on activation of the saccharide with 1-cyano-4-dimethylamino pyridinium tetrafluoroborate (CDAP) to form a cyanate ester. Such conjugates are described in PCT published application WO 93/15760 Uniformed Services University and WO 95/08348 and WO 96/29094. See also Chu C. et al Infect. Immunity, 1983 245 256.

### Ion exchange chromatography

Ion exchange chromatography techniques and principles are well known in the art, and are described in detail in standard textbookds such as Weiss, 'Handbook of Ion Chromatography', Wiley 2016, and in manufacturer's handbooks, for example 'Ion Exchange Chromatography Principles and Methods' from GE Healthcare (GE Healthcare Bio-Sciences AB, Uppsala, Sweden).

Ion exchange resins are composed of a base matrix, usually porous beads providing a wide adsorption surface, on which a charged ligand, usually a charged polymer to improve the resin's capacity, is immobilized. Exchanger resins are acid and bases themselves and their degree of protonation on a wide or narrow pH range depends on their being strong or weak acids or bases.

Ion exchange chromatography requires stationary phases characterised by mechanical stability, reduced aspecific adsorption, higher binding capacity and accelerated mass transfer. Stationary phases are typically composed of bead-shaped matrices comprising liquid-filled pores. Mechanically stable, functional matrices are commonly polysaccharides (cellulose, dextran, and agarose), synthetic organic polymers (polyacrylamide, polymethacrylate, polystyrene), and inorganic materials (silica, hydroxyapatite) which are chemically crosslinked and decorated with functional ligands. Their particle sizes range from 2 µm for analytical purposes up to about 200 µm for low-pressure preparative applications, whereas pore sizes are in the range of 10-100 nm.

As protein binding to exchange resin occurs at low salt concentration and elution occurs at high salt concentration, ion exchange chromatography columns should be washed with salt-containing buffer (suitably 1M NaCl) to entirely saturate the charged ligands before equilibrating with a buffer suitable to maintain protein solubility and stability. Protein loading is performed at a pH and conductivity as similar as possible to the equilibration buffer containing a low salt concentration to allow protein binding to exchangers. After loading, the unbound material is washed out, usually with equilibration buffer, possibly containing specific supplements. Elution can be performed by isocratic or gradient elution; gradient elution is preferred as it widens the elution window and can consist of linear or step salt gradient, usually consisting of a gradient of two buffers (equilibration buffer and buffer used for counterions loading). Alternatively, elution by pH gradient can be performed.

Typically, then, a step of ion exchange chromatography will involve the steps of
(i) binding the fusion protein to an ion exchange resin using a loading buffer,
(ii) washing the ion exchange resin using a washing buffer, and
(iii) eluting the protein from the ion exchange resin using an elution buffer.

The ion exchange resin may be a cation exchanger or an anion exchanger. A wide range of pre-prepared resins are commercially available, with different strengths and particle sizes. Commercially available cation exchange ('CIX') resins include Nuvia-S and Nuvia HR-S (Bio-Rad); Capto-S, Source 15S, CM Sephadex C-25 and CM-Sephadex C-50 (GE Healthcare). Commercially available anion exchange resins include Nuvia-Q and Nuvia HR-Q (Bio-Rad), Capto-Q, Source 15Q, DEAE Sephadex A-25 and DEAE-Sephadex A-50 (GE Healthcare). Strong cation exchange resins include Capto-S and Source 15S. Strong anion exchange resins include Capto-Q and Source 15Q. Weak cation exchange resins include CM Sephadex C-25 and CM-Sephadex C-50. Weak anion exchange resins includeDEAE Sephadex A-25 and DEAE-Sephadex A-50.

The composition of the equilibration, loading, washing and elution buffers may be selected by the skilled person in accordance with routine procedures in the art. Suitable buffers are well known in the art, as described in for example Weiss, 'Handbook of Ion Chromatography', Wiley 2016, and 'Ion Exchange Chromatography Principles and Methods' from GE Healthcare, described above. The choice of chromatographic buffer depends on the target protein pI, on its stability and solubility, but also on characteristics of the exchanger; buffers like Tris and acetate, which can bind exchangers should be avoided. Preferably 10-100 mM buffer concentration is recommended, corresponding to a conductivity of 1-4 mS/cm.

In an embodiment, the same buffer may be used for loading and washing, and the salt concentration then increased in the elution buffer. For example, 20 mM Citrate, 50 mM NaCl, pH 5.5 may be used for loading and washing, and elution then performed using 20 mM NaCitrate, , 50-500 mM NaCl, pH 5.5 .

The step of ion exchange chromatography may be repeated, optionally using a different ion exchange resin.

The step of ion exchange chromatography may be preceded or followed by additional purification steps, such as desalting or dialysis.

All references or patent applications cited within this patent specification are incorporated by reference herein.

Aspects of the invention are summarised in the following numbered paragraphs:
1. A fusion protein suitable for purification via ion exchange chromatography, which protein comprises
   (i) a protein of interest
   (ii) a peptide tag at the N or C terminus;
   wherein the peptide tag comprises Rₙ, (HR)ₙ, (PR)ₙ, (SR)ₙ or (PSR)ₙ, where 'n' is an integer from 2 to 6 inclusive.
2. A fusion protein comprising a protein of interest covalently linked directly or indirectly to a peptide tag which is capable of binding to an ion exchange resin, wherein the peptide tag comprises Rₙ, (HR)ₙ, (PR)ₙ, (SR)ₙ or (PSR)ₙ, where 'n' is an integer from 2 to 6 inclusive.
3. A fusion protein according to paragraph 1 or paragraph 2, wherein the peptide tag is from 2 to 20 amino acids in length.
4. A fusion protein according to paragraph 3, wherein the peptide tag is from 4 to 12 amino acids in length.
5. A fusion protein according to any one of paragraphs 1 to 4, wherein the peptide tag comprises an amino acid sequence of any one of SEQ ID Nos 1 to 9.
6. A fusion protein according to paragraph 5, wherein the peptide tag consists of an amino acid sequence of any one of SEQ ID Nos 1 to 9.
7. A fusion protein according to any one of paragraphs 1 to 6, further comprising a linker between the protein of interest and the peptide tag.
8. A fusion protein according to paragraph 7, wherein the linker comprises GG, GS, SS or SG.
9. A fusion protein according to any one of paragraphs 1 to 8, wherein the protein of interest is an antigenic protein or a carrier protein.
10. A fusion protein according to paragraph 9, wherein the protein of interest is tetanus toxoid (TT), diphtheria toxoid (DT), CRM₁₉₇, AcrA from *C. jejuni,* protein D from *Haemophilus influenzae,* exotoxin A of *Pseudomonas aeruginosa* (EPA), detoxified pneumolysin from *Streptococcus. pneumoniae,* meningococcal outer membrane protein complex (OMPC), detoxified Hla from *S. aureus* or ClfA from *S. aureus.*
11. A fusion protein according to paragraph 10, wherein the protein of interest is exotoxin A from *Pseudomonas aeruginosa* (EPA).
12. A fusion protein according to paragraph 11, wherein said EPA comprises the amino acid sequence of SEQ ID NO. 10 or an amino acid sequence at least 80%, 85%, 90%, 92%, 95%, 96%, 97%, 98% or 99% identical to SEQ ID NO. 10.
13. A fusion protein according to paragraph 11 or paragraph 12, wherein the EPA protein is modified in that
   a. it comprises a L to V substitution at the amino acid position corresponding to position L552 of SEQ ID NO. 10, and/or deletion of E553 of SEQ ID NO: 10, or at equivalent positions within an amino acid sequence at least 80%, 85%, 90%, 92%, 95%, 96%, 97%, 98% or 99% identical to SEQ ID NO. 10 (e.g. SEQ ID NO: 11).; and/or
   b. one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline, which substitution is optionally substitution with K-D-Q-N-R-T-K (SEQ ID NO: 27) or K-D-Q-N-A-T-K (SEQ ID NO: 28).
14. A fusion protein according to any one of paragraphs 11 to 13, wherein the protein of interest comprises the amino acid sequence of SEQ ID NO: 11 or an amino acid sequence at least 80%, 85%, 90%, 92%, 95%, 96%, 97%, 98% or 99% identical to SEQ ID NO. 11.
15. A fusion protein according to any one of paragraphs 1 to 14, wherein the fusion protein comprises (i) EPA as defined in any one of paragraphs 11 to 14, and (ii) a peptide tag as defined in any one of paragraphs 1 to 6.
16. A fusion protein according to paragraph 15, wherein the peptide tag comprises or consists of the amino acid sequence of any one of SEQ ID Nos: 6, 8 or 9.
17. A fusion protein according to paragraph 16, wherein the peptide tag comprises or consists of the amino acid sequence of SEQ ID No: 8.
18. A fusion protein according to paragraph 15, wherein the fusion protein comprises the amino acid sequence of any one of SEQ ID NOs: 12 to 18.
19. A fusion protein according to paragraph 15, wherein the fusion protein comprises the amino acid sequence of any one of SEQ ID NOs: 14, 17 or 18.
20. A fusion protein according to any one of paragraphs 1 to 8, wherein the protein of interest is Hla from *Staphylococcus aureus.*
21. A fusion protein according to paragraph 20, wherein said Hla comprises the amino acid sequence of SEQ ID NO. 19 or an amino acid sequence at least 80%, 85%, 90%, 92%, 95%, 96%, 97%, 98% or 99% identical to SEQ ID NO. 19.
22. A fusion protein according to paragraph 21, wherein the Hla protein is modified in that
   a. the amino acid sequence comprises an amino acid substitution at position H35 of SEQ ID NO. 19 or at an equivalent position within an amino acid sequence at least 80%, 85%, 90%, 92%, 95%, 96%, 97%, 98% or 99% identical to SEQ ID NO. 19, which substitution is optionally H35L;
   b. one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline, which substitution is optionally substitution of K131 of SEQ ID NO: 19 with K-D-Q-N-R-T-K (SEQ ID NO: 27); and/or
   c. the amino acid sequence comprises amino acid substitutions at positions H48 and G122 of SEQ ID NO. 19 or at equivalent positions within an amino acid sequence at least 80%, 85%, 90%, 92%, 95%, 96%, 97%, 98% or 99% identical to SEQ ID NO. 19, wherein said substitutions optionally are respectively H to C and G to C.
23. A fusion protein according to any one of paragraphs 20 to 22, wherein the protein of interest comprises the amino acid sequence of SEQ ID NO: 20 or an amino acid sequence at least 80%, 85%, 90%, 92%, 95%, 96%, 97%, 98% or 99% identical to SEQ ID NO. 20.
24. A fusion protein according to any one of paragraphs 1 to 8 or 20 to 23, wherein the fusion protein comprises (i) Hla as defined in any one of paragraphs 20 to 23, and (ii) a peptide tag as defined in any one of paragraphs 1 to 6.
25. A fusion protein according to paragraph 24, wherein the peptide tag comprises or consists of the amino acid sequence of any one of SEQ ID Nos: 1-3.
26. A fusion protein according to paragraph 25, wherein the peptide tag comprises or consists of the amino acid sequence of SEQ ID No: 1 or SEQ ID NO: 2.
27. A fusion protein according to paragraph 25, wherein the fusion protein comprises the amino acid sequence of any one of SEQ ID NOs: 21 to 23.
28. A fusion protein according to paragraph 15, wherein the fusion protein comprises the amino acid sequence of any one of SEQ ID NOs: 21 or 23.
29. A nucleic acid encoding a fusion protein according to any one of paragraphs 1 to 28.
30. An expression vector comprising a nucleic acid according to paragraph 29.
31. A host cell comprising a vector according to paragraph 30.
32. A protein-polysaccharide conjugate comprising a fusion protein according to any one of paragraphs 1 to 28 wherein the protein is conjugated to a polysaccharide to form a conjugate.
33. A conjugate according to clam 32, wherein the polysaccharide is a bacterial capsular polysaccharide.
34. A conjugate as according to paragraph 32 or paragraph 33, wherein the conjugate is a bioconjugate.
35. A method of purifying a fusion protein according to any one of paragraphs 1 to 28, or a conjugate of any one of paragraphs 32 to 34, the method comprising a step of ion exchange chromatography.
36. A method according to paragraph 35 wherein the peptide tag in said fusion protein serves to bind the fusion protein to the ion exchange resin.
37. A method of purifying a protein of interest, the method comprising (i) producing a fusion protein comprising the protein of interest and a peptide tag which binds to an ion exchange resin, and (ii) purifying the fusion protein by ion exchange chromatography.
38. A method of purification of a protein of interest comprising subjecting the protein to ion exchange chromatography, wherein the protein has been modified by addition of a peptide tag at the N or C terminus.
39. A method according to paragraph 37 or paragraph 38 wherein the peptide tag serves to bind the fusion protein to the ion exchange resin.
40. A method according to any one of paragraphs 37 to 39 wherein the peptide tag comprises Rₙ, (HR)ₙ, (PR)ₙ, (SR)ₙ or (PSR)ₙ.
41. A method according to paragraph 40, wherein 'n' is an integer from 2 to 6 inclusive.
42. A method according to any one of paragraphs 37 to 42, wherein the peptide tag is from 2 to 20 amino acids in length.
43. A method according to paragraph 42, wherein the peptide tag is from 4 to 12 amino acids in length.
44. A method according to any one of paragraphs 37 to 43, wherein the peptide tag comprises an amino acid sequence of any one of SEQ ID Nos 1 to 9.
45. A method according to any one of paragraphs 37 to 44, wherein the peptide tag consists of an amino acid sequence of any one of SEQ ID Nos 1 to 9.
46. A method according to any one of paragraphs 37 to 45, wherein said fusion protein further comprises a linker between the protein of interest and the peptide tag.
47. A method according to paragraph 46, wherein the linker comprises GG, GS, SS or SG.
48. A fusion protein according to any one of paragraphs 1 or 3-28, or a method according to any one of paragraphs 35-47, wherein the ion exchange chromatography is cation exchange chromatography.

### EXAMPLES

### Example 1 - Purification of Hla-CP5 carrying different tags on cation exchange column

### Sa5H Nuvia HR-S binding experiment

### Materials:

Nuvia HR-S CIX chromatography resin was obtained from BioRad (USA). Chemicals were obtained from Sigma-Aldrich (Switzerland) if not otherwise stated. Reaction tubes were obtained from TPP (Switzerland). Table top centrifuge was 5804 R (Eppendorf, Switzerland) was used. NuPAGE 4-12% BisTris SDS-PAGE Gels and coomassie safe stain were obtained from Invitrogen (USA). Plasmids encoding Hla with different C-terminal tags (HHHH, RRRR, HHRR and HRHR) and were ordered and obtained from Genecust (France).

### Methods:

*E. coli* strain W3110 was modified to produce S. aureus capsular polysaccharide CP5. This strain was transformed with a plasmid encoding pgIB (pGVXN1221) and the corresponding Hla encoding plasmid obtained from Genecust. Strains were grown in a 6-pack fermenter system in 2L vessels using complex medium containing yeast extract and soy peptone according to standard procedures. Arabinose and IPTG was used for induction of Hla and PgIB, respectively. Harvest was performed by centrifugation and cell pellets were frozen at -20°C until further use. Periplasmic extracts were obtained from cell pellets corresponding to 1 mL fermenter volume with an osmotic shock procedure. For this, cells were resuspended in a solution of 25% Sucrose, 100 mM EDTA, 200 mM Tris, pH8, incubated for 30 min on ice. To shock the cells, pellets obtained after centrifugation were resuspended in cold H2O. The supernatants were kept at RT until further use.

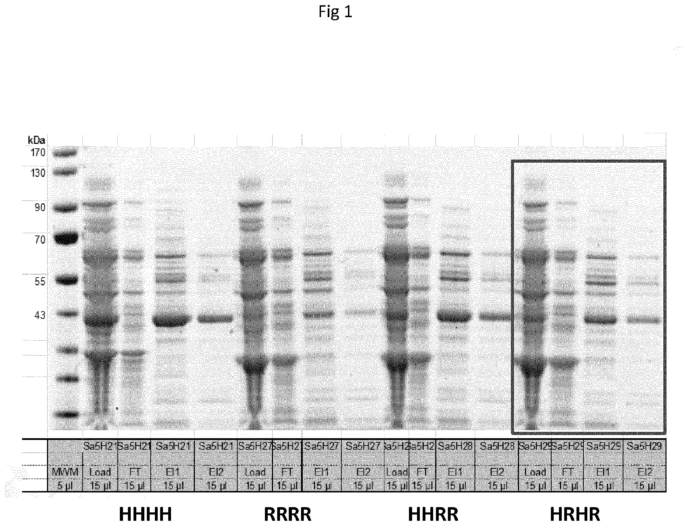

4 x 100µl of Nuvia HR-S chromatography resin were transferred to 4 x 15 ml TPP tubes. The tubes were centrifuged for 5 minutes at 2000 rpm. The supernatants were discarded. The beads were washed 2 times with 800 µl of Buffer A (20 mM NaCitrate, pH 5.5). 800 µl of the individual osmotic shock sample was diluted with 1.6ml BufferA and mixed with chromatography resin. The mixtures were incubated for 20 min at RT. The tubes were manually shaken 4-5 times during the incubation time. The supernatant of the centrifuged samples was labeled as flowthrough (FT). The beads were washed 3 times with 800 µl Buffer A. The wash fractions were discarded. Elution was performed by applying 2 times 300 µl Buffer B (20 mM NaCitrate, 500 mM NaCl, pH 5.5). Elution fractions were labeled as EL1 and EL2. FT and EL fractions were analyzed by SDS-PAGE using 4-12% BisTris Gels and staining with coomassie safe stain. The results are shown in Figure 1.

### Example 2: Purification of tagged (HRHR tag) and untagged Hla-CP5 using cationic exchange chromatography

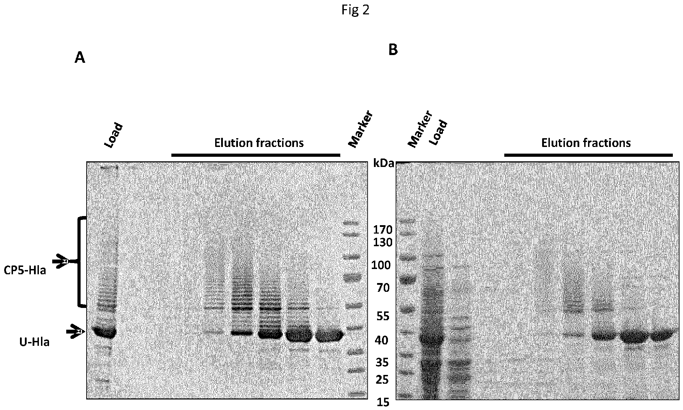

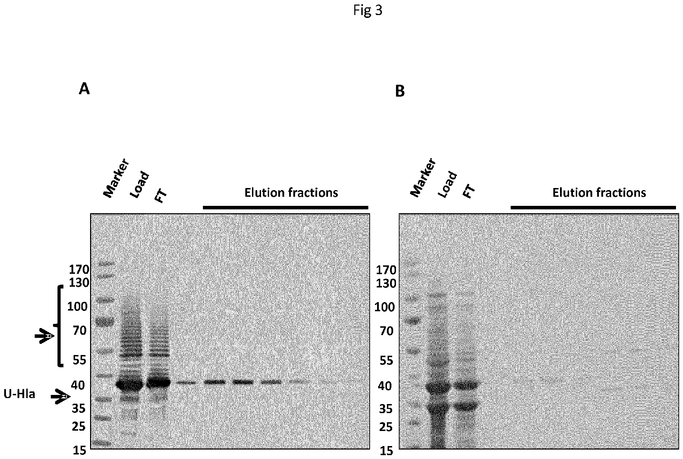

The HRHR tagged CP5-Hla bioconjugate was selected for a refinement of the selective purification step using a cationic exchange resin was performed, as shown in Figure 2. Results obtained using CP5-Hla lacking a purification tag are shown in Figure 3. StGVXN1717 (W3110 ΔwaaL; ΔwecA-wzzE; rmlB-wecG::Clm) was co-transformed with the plasmids encoding the *S.aureus* capsular polysaccharide CP5 (CPS 5) pGVXN393, the *S. aureus* carrier protein Hla_{H35L-H48C-G122C} pGVXN2533 carrying a glycosylation site at position 131, with or without a C-terminal histidine-arginine-histidine-arginine tag and *Campylobacter jejuni* oligosaccharyltransferase PgIB_{*cuo* **N311V-K482R-D483H-A669V**} pGVXN1221, by electroporation.

Briefly, cells were grown in TB medium, recombinant polysaccharide was expressed constitutively, Hla and PgIB were induced at an optical density OD₆₀₀ₙₘ of 0.74.

After overnight induction, cells were harvested and the CP5-Hla bioconjugate was released from the periplasm by an osmotic shock procedure. Cells were resuspended in 8.3mM Tris-HCI pH 7.4, 43.3mM NaCl, 0.9mM KCI and resuspension buffer (75% (w/v) sucrose, 30 mM EDTA, 600 mM Tris-HCI pH 8.5) and rotated for 20 minutes at 4°C. Cells were pelleted and resuspended in osmotic shock buffer (10 mM Tris-HCI pH 8.0) followed by another incubation of 20 minutes at 4°C. Cells were spun down again and the supernatant was loaded onto a 1 ml cation exchange column and the bioconjugate was recovered by a gradient elution. Proteins from the elution fractions were separated by a 4-12% SDS-PAGE and blotted onto a nitrocellulose membrane and detected by an anti-HIa antibody or the gel was directly stained with SimplyBlue Safe Stain. The results are shown in Figures 2 (with tag) and 3 (without tag).

In more detail: For the tagged protein, *E.coli* cells were harvested, spun down at 4°C, 9000rpm for 15 minutes and washed with 110 ml 0.9% sodium chloride and an equivalent of 1560 OD600nm were extracted by an osmotic shock procedure. Cells were resuspended in 5ml 1/3 x TBS (Tris buffered saline, Fisher Scientific) and 2.5ml resuspension buffer (75% (w/v) sucrose, 30 mM EDTA, 600 mM Tris-HCI pH 8.5) and rotated for 20 minutes at 4°C. Cells were pelleted and resuspended in 7.5ml osmotic shock buffer (10 mM Tris-HCI pH 8.0) followed by another incubation of 30 minutes at 4°C. Cells were spun down again by centrifugation, supernatants were recovered and filtered with a 0.2 micrometer filter. 2ml of the filtrate were supplemented with a 5M sodium chloride solution to a final concentration of 50mM and the pH was adjusted to 5.5 with 1M citric acid. The sample was spun down by centrifugation at 14000 rpm, at 4°C for 5 minutes. A purification column was prepared (Proteus FliQ FPLC column; 1ml; generon) with 1 ml of a cation exchange resin (Nuvia HR-S, Biorad) and equilibrated with 20 mM Citrate, 50 mM NaCl, pH 5.5 on an FPLC system (Aekta, Amersham Pharmacia). The sample was applied with a 2 ml superloop, the column was washed with 5 ml 20 mM Citrate, 50 mM NaCl, pH 5.5 and the bioconjugate was eluted applying a gradient to 20 mM Citrate, 500 mM NaCl, pH 5.5 in 10 column volumes. Flow-through and wash fractions collected were 500 microlitre, elution fractions had a volume of 350 microlitre. 45 microlitre of the chromatography fractions were supplemented with 15 microlitre 4 times concentrated Laemmli buffer to obtain a final concentration of 62.5mM Tris-HCI pH 6.8, 2% (w/v) sodium dodecyl sulfate, 5% (w/v) beta-mercaptoethanol, 10% (v/v) glycerol, 0.005% (w/v) bromphenol blue. Samples were boiled at 95°C for 15 minutes, 40 microlitres were separated by 4-12% SDS-PAGE (Nu-PAGE, 4-12% Bis-Tris Gel, life technologies) with MOPS running buffer (50 mM MOPS, 50 mM Tris Base, 0.1% SDS, 1 mM EDTA, pH 7.7) at 200 Volt for 45 minutes. Proteins were then transferred onto a nitrocellulose membrane using the iBLOT gel transfer stacks (Novex, by Life Technologies). The nitrocellulose was blocked with 10% (w/v) milk powder dissolved in PBST (10mM phosphate buffer pH 7.5, 137mM sodium chloride, 2.7mM potassium chloride purchased from Ambresco E703-500ml, 0.1% /v/v) tween) for 20 minutes at room temperature followed by an immunoblot detection using a primary rabbit anti-Hla antibody (polyclonal purified IgG, Glycovaxyn Nr 160) at 2.5 µg / ml in PBST for 1 hour at room temperature. The membrane was washed twice with PBST and incubated with a secondary goat anti-rabbit horse radish peroxidase (HRP) coupled antibody (Biorad, 170-6515) in PBST for 1 hour at room temperature. The membrane was washed 3 times with PBST for 5 minutes and protein bands were visualized by addition of TBM (TMB one component HRP membrane substrate) and the reaction was stopped with deionized water.

From the boiled samples, 20 microlitres were loaded on a second 4-12% SDS-PAGE gel (Nu-PAGE, 4-12% Bis-Tris Gel, life technologies) and proteins were separated in MOPS running buffer (50 mM MOPS, 50 mM Tris Base, 0.1% SDS, 1 mM EDTA, pH 7.7) at 200 Volt for 45 minutes. The gel was stained two consecutive times with 10 ml SimplyBlue SafeStain (Life Technologies) followed by a destaining step using deionized water. The results are shown in Figure 2.

For the non-tagged protein, *E.coli* cells were harvested, spun down at 4°C, 9000rpm for 15 minutes and washed with 110 ml 0.9% sodium chloride and an equivalent of 4200 OD600ₙₘ were extracted by an osmotic shock procedure. Cells were resuspended in 14ml 1/3 x TBS (Tris buffered saline, Fisher Scientific) and 7ml resuspension buffer (75% (w/v) sucrose, 30 mM EDTA, 600 mM Tris-HCI pH 8.5) and rotated for 30 minutes at 4°C. Cells were pelleted by centrifugation at 8000 rpm for 30 minutes at 4°C and resuspended in 21ml osmotic shock buffer (10 mM Tris-HCI pH 8.0) followed by another incubation of 30 minutes at 4°C. Cells were spun down again by centrifugation, supernatants were recovered and filtered with a 0.2 micrometer filter. 2ml of the filtrate were supplemented with a 5M sodium chloride solution to a final concentration of 50mM, the pH was set to 5.5 with 1M citric acid by adjusting the volume to 4 ml. The sample was spun down by centrifugation at 14000 rpm, at 4°C for 5 minutes. A purification column was prepared (Proteus FliQ FPLC column; 1ml; generon) with 1 ml of a cation exchange resin (Nuvia HR-S, Biorad) and equilibrated with 20 mM Citrate, 50 mM NaCl, pH 5.5 on an FPLC system (Aekta, Amersham Pharmacia). 2ml of the sample was applied with a 2 ml superloop, the column was washed with 5 ml 20 mM Citrate, 50 mM NaCl, pH 5.5 and the bioconjugate was eluted applying a gradient to 20 mM Citrate, 500 mM NaCl, pH 5.5 in 10 column volumes. Flow-through and wash fractions collected were 500 microliter, elution fractions had a volume of 350 microliter. 45 microliter of the chromatography fractions were supplemented with 15 microliter 4 times concentrated Laemmli buffer to obtain a final concentration of 62.5mM Tris-HCI pH 6.8, 2% (w/v) sodium dodecyl sulfate, 5% (w/v) beta-mercaptoethanol, 10% (v/v) glycerol, 0.005% (w/v) bromphenol blue. Samples were boiled at 95°C for 15 minutes. 20 microliters thereof were separated by 4-12% SDS-PAGE (Nu-PAGE, 4-12% Bis-Tris Gel, life technologies) with MOPS running buffer (50 mM MOPS, 50 mM Tris Base, 0.1% SDS, 1 mM EDTA, pH 7.7) at 200 Volt for 45 minutes for the Western Blot shown in Figure 3) A). Proteins were then transferred onto a nitrocellulose membrane using the iBLOT gel transfer stacks (Novex, by Life Technologies). The nitrocellulose was blocked with 10% (w/v) milk powder dissolved in PBST (10mM phosphate buffer pH 7.5, 137mM sodium chloride, 2.7mM potassium chloride purchased from Ambresco E703-500ml, 0.1% /v/v) tween) for 20 minutes at room temperature followed by an immunoblot detection using a primary rabbit anti-Hla antibody (polyclonal purified IgG, Glycovaxyn Nr 160) at 2.5 ug / ml in PBST for 1 hour at room temperature. The membrane was washed twice with PBST and incubated with a secondary goat anti-rabbit horse radish peroxidase (HRP) coupled antibody (Biorad, 170-6515) in PBST for 1 hour at room temperature. The membrane was washed 3 times with PBST for 5 minutes and protein bands were visualized by addition of TBM (TMB one component HRP membrane substrate) and the reaction was stopped with deionized water.

From the boiled samples, 40 microliters were loaded on a second 4-12% SDS-PAGE gel for SimplyBlues staining (Nu-PAGE, 4-12% Bis-Tris Gel, life technologies) and proteins were separated in MOPS running buffer (50 mM MOPS, 50 mM Tris Base, 0.1% SDS, 1 mM EDTA, pH 7.7) at 200 Volt for 45 minutes. The gel was stained two consecutive times with 10 ml SimplyBlue SafeStain (Life Technologies) followed by a destaining step using deionized water. The results are shown in Figure 3, and show that the untagged protein did not bind to the ion exchange resin, unlike the tagged protein.

### Example 3 Purification of tagged EPA bioconjugates using Nuvia-S and Capto-S ion exchange chromatography

### Materials:

Modified EPA was tested with following resins: Nuvia S (BioRad), Capto S Impact (GE Healthcare). NGC System from BioRad was used. Buffer composition: Sodium-Acetate or Sodium Phosphate, Sodium and Sodium Chloride (Sigma). IPC SDS-PAGE and Coomassie save stain were done as described above. Western Blot: Rabbit Antibody anti EPA was obtained from Sigma P2318 and goat anti rabbit HRP Antibody from Biorad 170-6515.

### Methods:

*E. coli* strain W3110 was modified to produce *S. pneumoniae* polysaccharides of serotype Sp33F. These strains were transformed with a plasmid encoding pgIB and the corresponding EPA encoding plasmid obtained from Genecust. After the fermentation the osmotic shock and clarification were performed as described above. The supernatant after centrifugation corresponded to the clarified lysate.

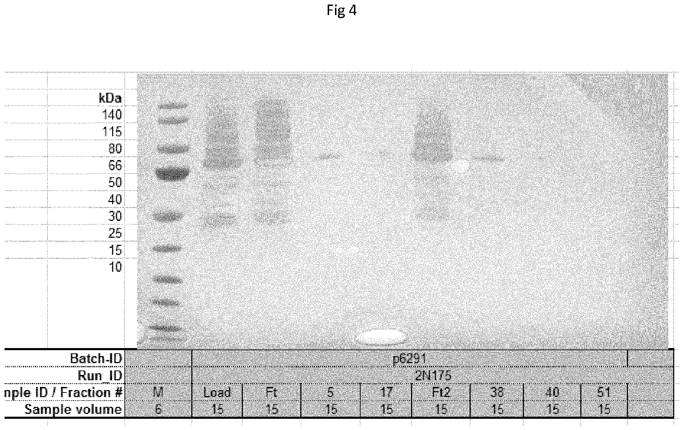

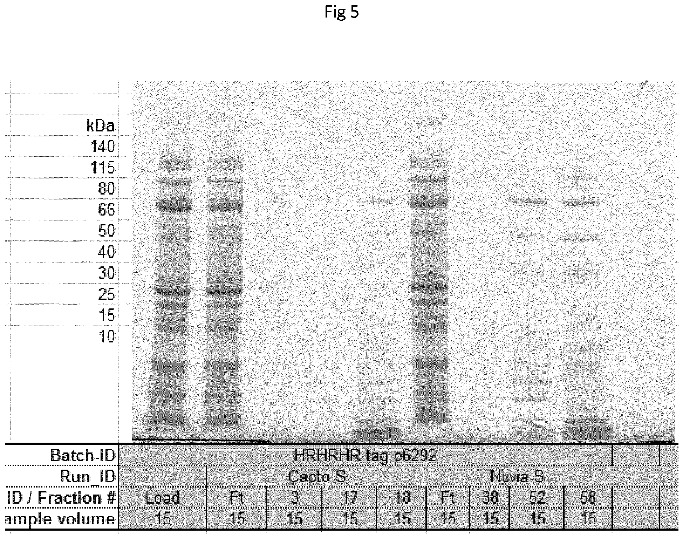

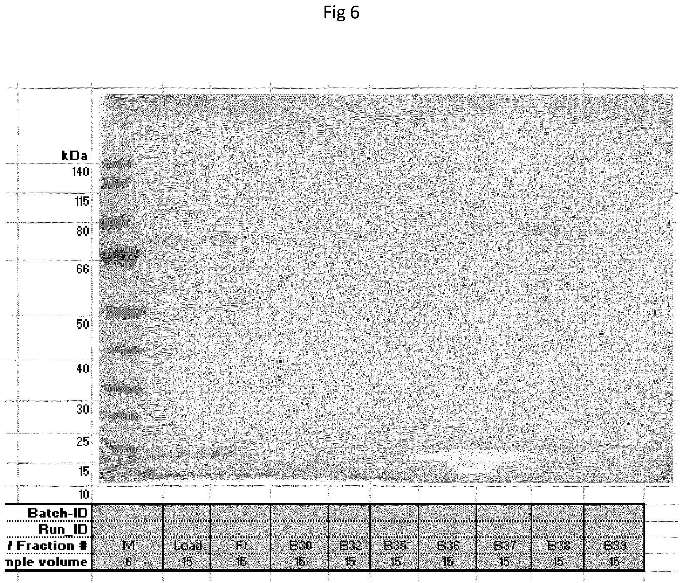

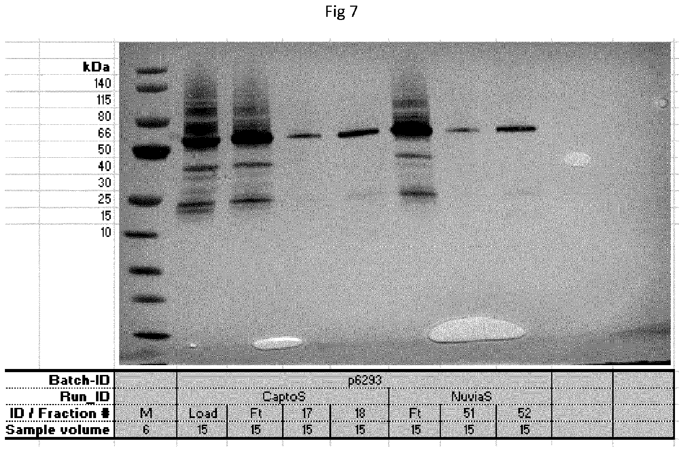

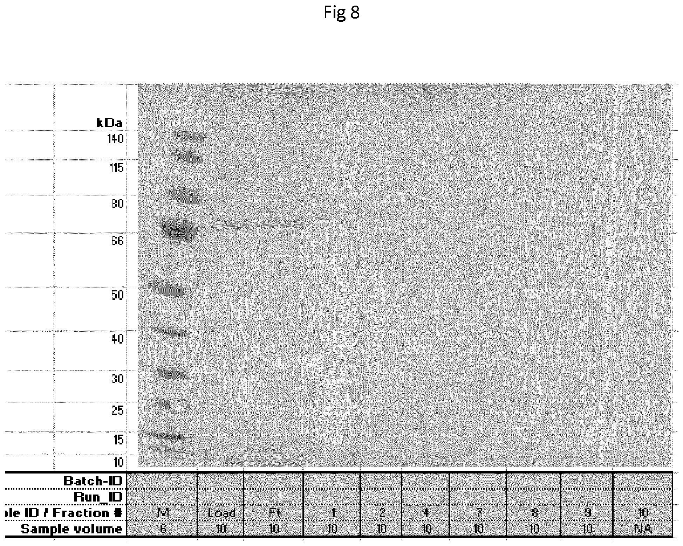

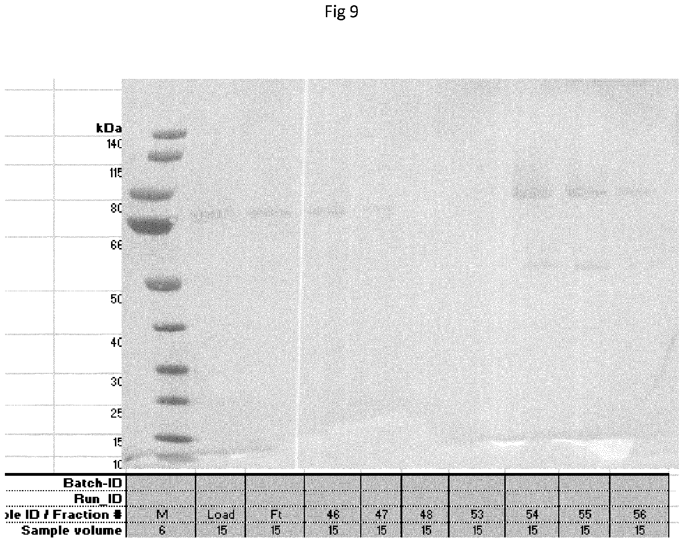

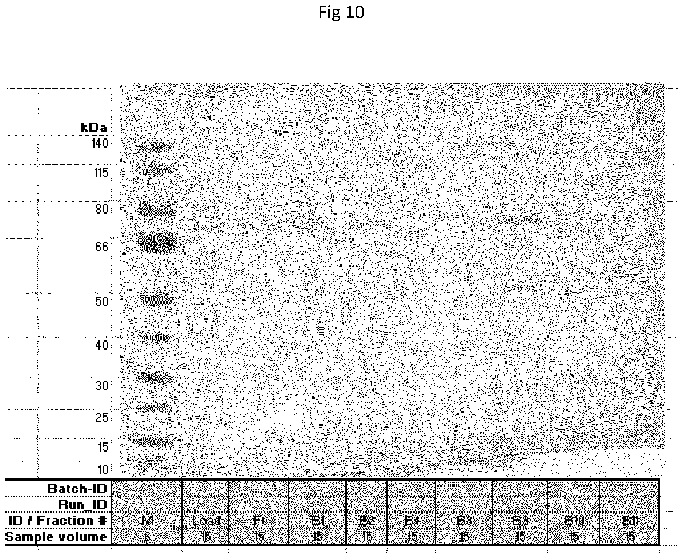

The pH of the clarified lysates containing glycosylated EPA with different peptide tags (HRHR, HRHRHR, HRHRHRHR, RRRR, RRRRRR, PRPRPRPRPRPR and PSRPSRPSRPSR) was adapted to pH 6.0±0.2 and loaded onto a Nuvia S or Capto S Impact column that previously had been equilibrated with 20mM Na-Acetate or NaPO4 both pH 5.8. A wash phase of 6 column volumes (CV) followed by 6 CV elution buffer (20mM Na-Acetate or NaPO4; 200mM SodiumChloride pH 6.0) was performed. The resin Capto S Impact showed enhanced capacity and efficacy and therefore was used for upscale from 5 mL to 100 mL column volume. Specific fractions of chromatography steps were analyzed by SDS PAGE and coomassie stained. Additionally, EPA specific Western Blots were performed to increase specificity and sensitivity. The results are shown in Figures 4-10. As can be seen, the best results were obtained for EPA-PRPRPRPRPRPR and EPA-PSRPRPSRPSR. R repeat tags and the shorter HR tags were not very effective, but the longest HR tag (HRHRHRHRHR) did bind to the column.

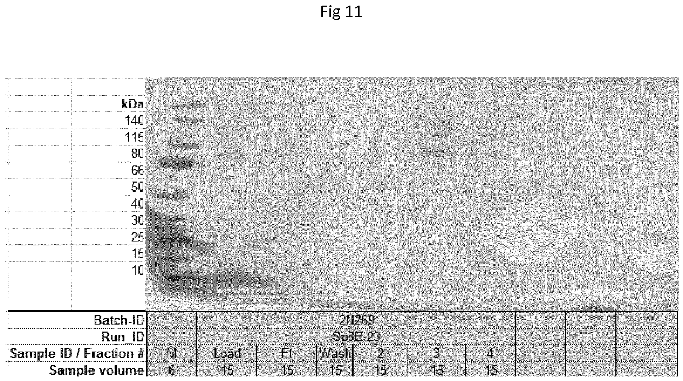

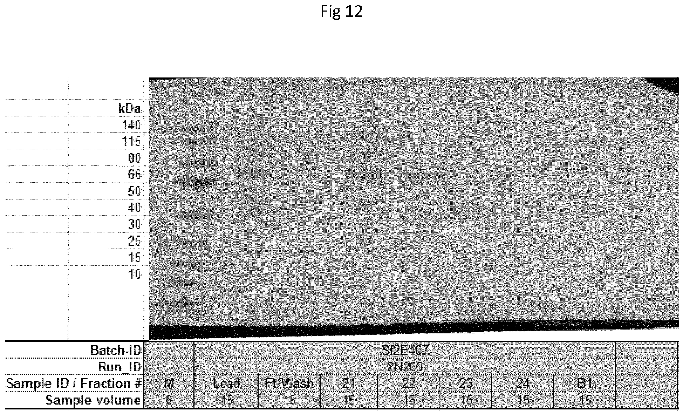

EPA-PRPRPRPRPRPR was also expressed in *E. coli* expressing *S pneumoniae* capsular polysaccharides from serotypes Sp8 and the *S. flexneri* 2aO polysaccharide to produce Sp8-EPA and Sf2-EPA bioconjugates, in order to test whether the conjugation of different PS affected the binding of EPA to the column Sp8 is negatively charged and 2a O is non-charged). The results are shown in Figures 11 and 12, which show that both the EPA-Sp8 and EPA-Sf2 still bound to Capto S.

### SEQUENCE LISTINGS

SEQ ID NO:1 Amino acid sequence of H4 tag
   HHHH
SEQ ID NO:2 Amino acid sequence of R4 tag
   RRRR
SEQ ID NO:3 Amino acid sequence of H2R2 tag
   HHRR
SEQ ID NO:4 Amino acid sequence of (HR)2 tag
   HRHR
SEQ ID NO:5 Amino acid sequence of (HR)3 tag
   HRHRHR
SEQ ID NO:6 Amino acid sequence of (HR)4 tag
   HRHRHRHR
SEQ ID NO:7 Amino acid sequence of R6 tag
   RRRRRR
SEQ ID NO:8 Amino acid sequence of (PR)6 tag
   PRPRPRPRPRPR
SEQ ID NO:9 Amino acid sequence of (PSR)4 tag
   PSRPSRPSRPSR
SEQ ID NO:10 Amino acid sequence of mature wild-type EPA. Bold and underlined are the residues substituted/removed for detoxification. 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRLETILGWPLAERTVVIPSAIPTDPRNVGGDLDPSSIPDKEQ
AISALPDYASQPGKPPREDLK
SEQ ID NO:11 Amino acid sequence of EPA with L552V/ΔE553 detoxifying mutation (bold, underlined) 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLK
SEQ ID NO:12 Amino acid sequence of EPA with detoxifying mutation and HR2 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKHRHR
SEQ ID NO:13 Amino acid sequence of EPA with detoxifying mutation and HR3 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
 
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKHRHRHR
SEQ ID NO:14 Amino acid sequence of EPA with detoxifying mutation and HR4 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKHRHRHRHR
SEQ ID NO:15 Amino acid sequence of EPA with detoxifying mutation and R4 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKRRRR
SEQ ID NO:16 Amino acid sequence of EPA with detoxifying mutation and R6 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKRRRRRR
SEQ ID NO:17 Amino acid sequence of EPA with detoxifying mutation and PR6 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKPRPRPRPRPRPR
SEQ ID NO:18 Amino acid sequence of EPA with detoxifying mutation and PSR4 tag 
AEEAFDLWNECAKACVLDLKDGVRSSRMSVDPAIADTNGQGVLHYSMVLEGGNDALKLAIDNALSITSDGLTIR
LEGGVEPNKPVRYSYTRQARGSWSLNWLVPIGHEKPSNIKVFIHELNAGNQLSHMSPIYTIEMGDELLAKLARD
ATFFVRAHESNEMQPTLAISHAGVSVVMAQAQPRREKRWSEWASGKVLCLLDPLDGVYNYLAQQRCNLDDTWEG
KIYRVLAGNPAKHDLDIKPTVISHRLHFPEGGSLAALTAHQACHLPLEAFTRHRQPRGWEQLEQCGYPVQRLVA
LYLAARLSWNQVDQVIRNALASPGSGGDLGEAIREQPEQARLALTLAAAESERFVRQGTGNDEAGAASADVVSL
TCPVAAGECAGPADSGDALLERNYPTGAEFLGDGGDVSFSTRGTQNWTVERLLQAHRQLEERGYVFVGYHGTFL
EAAQSIVFGGVRARSQDLDAIWRGFYIAGDPALAYGYAQDQEPDARGRIRNGALLRVYVPRWSLPGFYRTGLTL
AAPEAAGEVERLIGHPLPLRLDAITGPEEEGGRVTILGWPLAERTVVIPSAIPTDPRNVGGDLDPSSIPDKEQA
ISALPDYASQPGKPPREDLKPSRPSRPSRPSR
SEQ ID NO:19 Amino acid sequence of mature wild-type Hla 
ADSDINIKTGTTDIGSNTTVKTGDLVTYDKENGMHKKVFYSFIDDKNHNKKLLVIRTKGTIAGQYRVYSEEGAN
KSGLAWPSAFKVQLQLPDNEVAQISDYYPRNSIDTKEYMSTLTYGFNGNVTGDDTGKIGGLIGANVSIGHTLKY
VQPDFKTILESPTDKKVGWKVIFNNMVNQNWGPYDRDSWNPVYGNQLFMKTRNGSMKAADNFLDPNKASSLLSS
GFSPDFATVITMDRKASKQQTNIDVIYERVRDDYQLHWTSTNWKGTNTKDKWIDRSSERYKIDWEKEEMTN
SEQ ID NO:20 Amino acid sequence of Hla with glycosite KDQNRTK substituted for K131, H35L detoxifying mutation, H48C/G122C stabilizing mutations (bold, underlined) 
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
SSLLSSGFSPDFATVITMDRKASKQQTNIDVIYERVRDDYQLHWTSTNWKGTNTKDKWIDRSSERYKIDWEKEE
MTN
SEQ ID NO:21 Amino acid sequence of Hla with glycosite, detoxifying and stabilizing mutations, linker and H4 tag 
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
SSLLSSGFSPDFATVITMDRKASKQQTNIDVIYERVRDDYQLHWTSTNWKGTNTKDKWIDRSSERYKIDWEKEE
MTNGSHHHH
SEQ ID NO:22 Amino acid sequence of Hla with glycosite, detoxifying and stabilizing mutations, linker and R4 tag 
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
SSLLSSGFSPDFATVITMDRKASKQQTNIDVIYERVRDDYQLHWTSTNWKGTNTKDKWIDRSSERYKIDWEKEE
MTNGSRRRR
SEQ ID NO:23 Amino acid sequence of Hla with glycosite, detoxifying and stabilizing mutations, linker and H2R2 tag 
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
 
SSLLSSGFSPDFATVITMDRKASKQQTNIDVIYERVRDDYQLHWTSTNWKGTNTKDKWIDRSSERYKIDWEKEE
MTNGSHHRR
SEQ ID NO:24 Amino acid sequence of Hla with glycosite, detoxifying and stabilizing mutations, linker and (HR)2 tag 
ADSDINIKTGTTDIGSNTTVKTGDLVTYDKENGMLKKVFYSFIDDKNCNKKLLVIRTKGTIAGQYRVYSEEGAN
KSGLAWPSAFKVQLQLPDNEVAQISDYYPRNSIDTKEYMSTLTYGFNCNVTGDDTGKDQNRTKIGGLIGANVSI
GHTLKYVQPDFKTILESPTDKKVGWKVIFNNMVNQNWGPYDRDSWNPVYGNQLFMKTRNGSMKAADNFLDPNKA
SSLLSSGFSPDFATVITMDRKASKQQTNIDVIYERVRDDYQLHWTSTNWKGTNTKDKWIDRSSERYKIDWEKEE
MTNGSHRHR
SEQ ID NO: 25 - Minimal PgIB glycosite consensus sequence
   D/E-X-N-Z-S/T
SEQ ID NO: 26 - Full PgIB glycosite consensus sequence
   K-D/E-X-N-Z-S/T-K
SEQ ID NO: 27 -PgIB glycosite sequence (Hla)
   KDNQNRTK
SEQ ID NO: 28 - PgIB glycosite sequence (EPA)
   KDNQNATK
SEQ ID NO: 29 - FlgI signal sequence
   MIKFLSALILLLVTTAAQA
SEQ ID NO: 30 - OmpA signal sequence
   MKKTAIAIAVALAGFATVAQA
SEQ ID NO: 31 - MalE signal sequence
   MKIKTGARILALSALTTMMFSASALA
SEQ ID NO: 32 - PelB signal sequence
   MKYLLPTAAAGLLLLAAQPAMA
SEQ ID NO: 33 - LTIIb signal sequence
   MSFKKIIKAFVIMAALVSVQAHA
SEQ ID NO: 34 - XynA signal sequence
   MFKFKKKFLVGLTAAFMSISMFSATASA
SEQ ID NO: 35 - DsbA signal sequence
   MKKIWLALAGLVLAFSASA
SEQ ID NO: 36 - TolB signal sequence
   MKQALRVAFGFLILWASVLHA
SEQ ID NO: 37 - SipA signal sequence
   MKMNKKVLLTSTMAASLLSVASVQAS

## Claims

1. A fusion protein suitable for purification via ion exchange chromatography, which protein comprises
(iii) a protein of interest
(iv) a peptide tag at the N or C terminus;
wherein the peptide tag comprises Rₙ, (HR)ₙ, (PR)ₙ, (SR)ₙ or (PSR)ₙ, where 'n' is an integer from 2 to 6 inclusive.

2. A fusion protein according to claim 1, wherein the peptide tag is from 2 to 20 amino acids in length, optionally 4 to 12 amino acids in length.

3. A fusion protein according to claim 1 or claim 2, wherein the peptide tag comprises or consists of an amino acid sequence of any one of SEQ ID Nos 1 to 9.

4. A fusion protein according to any one of claims 1 to 3, further comprising a linker between the protein of interest and the peptide tag, wherein the linker optionally comprises GG, GS, SS or SG.

5. A fusion protein according to any one of claims 1 to 4, wherein the protein of interest is an antigenic protein or a carrier protein, wherein the protein of interest is tetanus toxoid (TT), diphtheria toxoid (DT), CRM₁₉₇, AcrA from *C. jejuni,* protein D from *Haemophilus influenzae,* exotoxin A of *Pseudomonas aeruginosa* (EPA), detoxified pneumolysin from *Streptococcus. pneumoniae,* meningococcal outer membrane protein complex (OMPC), detoxified Hla from *S. aureus* or ClfA from *S. aureus.*

6. A fusion protein according to claim 5, wherein the protein of interest is EPA, wherein said EPA optionally comprises the amino acid sequence of SEQ ID NO. 10 or an amino acid sequence at least 80%, 85%, 90%, 92%, 95%, 96%, 97%, 98% or 99% identical to SEQ ID NO. 10;
wherein the EPA protein is optionally modified in that
a. it comprises a L to V substitution at the amino acid position corresponding to position L552 of SEQ ID NO. 10, and/or deletion of E553 of SEQ ID NO: 10, or at equivalent positions within an amino acid sequence at least 80%, 85%, 90%, 92%, 95%, 96%, 97%, 98% or 99% identical to SEQ ID NO. 10 (e.g. SEQ ID NO: 11).; and/or
b. one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline, which substitution is optionally substitution with K-D-Q-N-R-T-K (SEQ ID NO: 27) or K-D-Q-N-A-T-K (SEQ ID NO: 28);
wherein the EPA protein optionally comprises the amino acid sequence of SEQ ID NO: 11 or an amino acid sequence at least 80%, 85%, 90%, 92%, 95%, 96%, 97%, 98% or 99% identical to SEQ ID NO. 11.

7. A fusion protein according to any one of claims 1 to 6, wherein the fusion protein comprises
(i) EPA as defined in any one of claims 9 to 12, and (ii) a peptide tag as defined in any one of claims 1 to 3; wherein the peptide tag optionally comprises or consists of the amino acid sequence of any one of SEQ ID Nos: 6, 8 or 9;
wherein the fusion protein optionally comprises the amino acid sequence of any one of SEQ ID NOs: 12 to 18, optionally SEQ ID NOs: 14, 17 or 18.

8. A fusion protein according to any one of claims 1 to 5, wherein the protein of interest is Hla from *Staphylococcus aureus,* wherein said Hla optionally comprises the amino acid sequence of SEQ ID NO. 19 or an amino acid sequence at least 80%, 85%, 90%, 92%, 95%, 96%, 97%, 98% or 99% identical to SEQ ID NO. 19;
wherein the Hla protein is optionally modified in that
a. the amino acid sequence comprises an amino acid substitution at position H35 of SEQ ID NO. 19 or at an equivalent position within an amino acid sequence at least 80%, 85%, 90%, 92%, 95%, 96%, 97%, 98% or 99% identical to SEQ ID NO. 19, which substitution is optionally H35L;
b. one or more amino acids have been substituted by one or more consensus sequence(s) selected from: D/E-X-N-Z-S/T (SEQ ID NO. 25) and K-D/E-X-N-Z-S/T-K (SEQ ID NO. 26), wherein X and Z are independently any amino acid apart from proline, which substitution is optionally substitution of K131 of SEQ ID NO: 19 with K-D-Q-N-R-T-K (SEQ ID NO: 27); and/or
c. the amino acid sequence comprises amino acid substitutions at positions H48 and G122 of SEQ ID NO. 19 or at equivalent positions within an amino acid sequence at least 80%, 85%, 90%, 92%, 95%, 96%, 97%, 98% or 99% identical to SEQ ID NO. 19, wherein said substitutions optionally are respectively H to C and G to C;
wherein said Hla optionally comprises the amino acid sequence of SEQ ID NO: 20 or an amino acid sequence at least 80%, 85%, 90%, 92%, 95%, 96%, 97%, 98% or 99% identical to SEQ ID NO. 20.

9. A fusion protein according to any one of claims 1 to 5 or 8, wherein the fusion protein comprises (i) Hla as defined in claim 8, and (ii) a peptide tag as defined in any one of claims 1 to 6, wherein the peptide tag optionally comprises or consists of the amino acid sequence of any one of SEQ ID Nos: 1-3, optionally the amino acid sequence of SEQ ID No: 1 or SEQ ID NO: 2;
wherein the fusion protein optionally comprises the amino acid sequence of any one of SEQ ID NOs: 21 to 24, optionally SEQ ID NOs: 21 or 23.

10. A nucleic acid encoding a fusion protein according to any one of claims 1 to 9, an expression vector comprising said nucleic acid, or a host cell comprising said vector.

11. A protein-polysaccharide conjugate comprising a fusion protein according to any one of claims 1 to 9 wherein the protein is conjugated to a polysaccharide, optionally a bacterial polysaccharide, to form a conjugate, optionally a bioconjugate.

12. A method of purifying a fusion protein according to any one of claims 1 to 9, or a conjugate of claim 11, the method comprising a step of ion exchange chromatography.

13. A method of purifying a protein of interest, the method comprising (i) producing a fusion protein comprising the protein of interest and a peptide tag which binds to an ion exchange resin, and (ii) purifying the fusion protein by ion exchange chromatography, wherein the peptide tag is optionally as defined in any one of claims 1 to 4.

14. A fusion protein according to any one of claims 1 to 9, or a method according to claim 12 or claim 13, wherein the ion exchange chromatography is cation exchange chromatography.
